**Europäisches Patentamt**

**European Patent Office**

**Office européen des brevets**

(11) Veröffentlichungsnummer: **0 047 492**
A2

# EUROPÄISCHE PATENTANMELDUNG

(21) Anmeldenummer: **81106871.7**

(22) Anmeldetag: **03.09.81**

(51) Int. Cl.³: **A 61 L 15/03**

(30) Priorität: **06.09.80 DE 3033606**

(43) Veröffentlichungstag der Anmeldung: **17.03.82**
**Patentblatt 82/11**

(84) Benannte Vertragsstaaten: **AT BE CH DE FR GB IT LI LU NL SE**

(71) Anmelder: **Intermedicat GmbH, Gerliswilstrasse 45, CH-6020 Emmenbrücke (CH)**

(72) Erfinder: **Dedem, Manfred, Dr., Im Kleinfeldchen 14b, D-6234 Eschborn 2 (DE)**
Erfinder: **Schröder, Jürgen, Dr., Auf dem Rottheil 7, D-3509 Spangenberg (DE)**

(74) Vertreter: **von Kreisler, Alek et al, Deichmannhaus am Hauptbahnhof, D-5000 Köln 1 (DE)**

(54) **Antiadhäsiver, antimikrobieller Wundverband und Verfahren zu dessen Herstellung.**

(57) Ein antiadhäsiver, antimikrobieller Wundverband auf der Basis von Verbandmaterial und pharmazeutischen Zubereitungen ist dadurch gekennzeichnet, dass ein antiadhäsiver oder antiadhäsiv ausgerüstetes Gewebe oder Vlies aus natürlichen oder synthetischen Fasern mit einer antimikrobiellen Zubereitung zur lokalen Therapie beschichtet ist.

EP 0 047 492 A2

VON KREISLER   SCHÖNWALD   EISHOLD   FUES

VON KREISLER   KELLER   SELTING   WERNER

PATENTANWÄLTE

Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

DEICHMANNHAUS AM HAUPTBAHNHOF

D-5000 KÖLN 1

0047492

B.Braun Melsungen AG

Melsungen

3.9.81     AvK/IM

Antiadhäsiver, antimikrobieller Wundverband und
Verfahren zu dessen Herstellung

-------------------------------------------------

Die Behandlung großflächiger, offener, nässender und evtl. infizierter
oder infektionsgefährdeter Wunden ist auch heute ein ungelöstes Problem.
Übliches Verbandmaterial verklebt oftmals mit der Wunde, so daß die
Entfernung eines Verbandes nicht nur für den Patienten äußerst
schmerzhaft ist, sondern durch erneutes Aufreißen der eingetrockneten,
verklebten Sekrete der Wundheilungsprozeß auch nachhaltig gestört wird.
Nach dem Stand der Technik bemüht man sich daher, wenn möglich,
antiadhäsive Verbände aus verschiedenen natürlichen oder synthetischen
oder entsprechend antiadhäsiv ausgerüstete Gewebe als Verbandmaterial
einzusetzen. Insbesondere bei großflächigen Wunden ist jedoch oftmals
der Einsatz dieser Gewebe nicht möglich, da derartige Wunden sehr
häufig infiziert sind und daher der lokalen antimikrobiellen Behandlung bedürfen. Für eine derartige lokale antimikrobielle Behandlung
großflächiger Wunden stehen nach dem Stand der Technik Wundverbände
zur Verfügung, welche mit geeigneten antimikrobiellen Salben vorzugsweise auf der Basis von PVP-Jod, ausgerüstet sind. Diese nach dem
Stand der Technik bekannten Wundverbände haben jedoch den Nachteil,
nicht antiadhäsiv zu sein, wodurch sich Verklebungen mit der Wunde
mit den damit verbundenen gravierenden Nachteilen ergeben.

Es war deshalb notwendig, wesentlich Besseres zu finden. Die Problemstellung ist daher, einen antiadhäsiven nicht mit der Wunde verklebenden und trotzdem antimikrobiell wirkenden Wundverband zu finden.

Gelöst wurde sie durch die Beschichtung eines antiadhäsiven oder antiadhäsiv ausgerüsteten Trägermaterials (Gewebe- oder Vliesstoff) aus natürlichen oder synthetischen Fasern mit einer antibakteriellen Zubereitung, z.B. einer Salbe.

Dementsprechend betrifft die Erfindung einen antiadhäsiven, antimikrobiellen Wundverband auf der Basis von Verbandstoffen und pharmazeutischen Zubereitungen, der dadurch gekennzeichnet ist, daß ein antiadhäsives oder antiadhäsiv ausgerüstetes Gewebe oder Vlies aus natürlichen oder synthetischen Fasern mit einer antimikrobiellen Zubereitung zur lokalen Therapie beschichtet ist.

Als antibakterielle Zubereitungen kommen vorzugsweise Salben, und zwar beispielsweise Wundsalben, Brandsalben, Heilsalben u.dgl. zur Verwendung. Als solche Salben können z.B. PVP-Jodsalbe (PVP-Jod ist die allgemein übliche Ab-kürzung für antimikrobiell hochwirksame Anlagerungsver-bindungen von Jod an Polyvinylpyrrolidon; s.z.B. USP XIX) oder Silbersulfadiazin-Salbe verwendet werden. Besonders bevorzugt wird PVP-Jodsalbe.

Anstelle von fetthaltigen Salben können ebensogut fett - freie Cremes verwendet werden.

Die Salbengrundlagen sind allgemein bekannt und im Handel erhältlich. Als Beispiele seien Polyäthylenglyko. oder äthoxylierte Fettalkohole genannt.

Als Gewebe oder Vliesstoffe kommen alle für den Anwendungszweck geeigneten Stoffe aus natürlichen oder synthetischen Fasern infrage. Als Beispiele seien Vliesstoffe
aus Cellulose oder aus Polytetrafluoräthylen (PTFE) sowie
grobmaschige Baumwollgewebe oder andere übliche Verbandstoffe genannt.

Die Erfindung umfasst ferner ein Verfahren zur Herstellung
der antiadhäsiven, antimikrobiellen Wundverbände. Bei
diesem Verfahren wird ein Vliesstoff oder ein Baumwollgewebe, das die Spezifikation des Deutschen Arznei-Buchs
(DAB) für Verbandmaterial erfüllt, durch Tauchen in ein Bad
aus heisser, verflüssigter Vaseline getränkt,
derart antiadhäsiv ausgerüsteten Gewebe oder Vliese werden
dann z.B. aus einer Breitschlitzdüse mit einer geeigneten
pharmazeutischen Zubereitung, z.B. einer PVP-Jod-Salbe
beschichtet. Anschließend werden die beschichteten Bahnen
auf die gewünschte Größe zerschnitten und in eine Verpackungsfolie eingesiegelt. Die Herstellung kann voll kontinuierlich ablaufen, indem ein Endloswundverband hergestellt
wird, indem das Gewebe oder der Vliesstoff von einer Rolle
abgewickelt wird, durch ein Bad mit z.B. geschmolzener
Vaseline gezogen, dann durch eine Abkühlzone geführt und
anschließend mit der geeigneten pharmazeutischen Zubereitung, z.B. der PVP-Jod-Salbe beschichtet oder bestrichen
wird.

Das Trägermaterial, d.h. der Gewebe- oder der Vliesstoff,
kann als solches bereits antiadhäsiv sein. Als Beispiele
seien Polytetrafluoräthylen oder Polyäthylen genannt.

Andere Trägermaterialien werden antiadhäsiv ausgerüstet,
indem sie z.B. mit Vaseline oder mit Paraffin oder ähnlichen
für Verbandmaterial zulässigen antiadhäsiv machenden Stoffen
getränkt werden.

Beim fertigen Wundverband gemäss der Erfindung überzieht die
pharmazeutische antimikrobielle Zubereitung, z.B. eine
antibakterielle Salbe, das durch das Trägermaterial gebildete lockere Gitterwerk mit einem zusammenhängenden Film, z.B. einem Salbenfilm. Beim Auflegen auf die Wunde bilden sich daher "Fenster", in denen die antimikrobielle Salbe in direktem Kontakt zur offenen Wunde steht. Durch das antiadhäsive Trägermaterial kann die sich zum Teil durch das Wundsekret verflüssigende Salbe nicht so schnell ablaufen wie bei einer üblichen dem Stand der Technik entsprechenden antimikrobiell ausgerüsteten Wundauflage. Dadurch ergeben sich die sehr erwünschten Effekte , daß die antimikrobielle Salbe auf der Wunde in höherer Konzentration vorhanden ist und daher besser und schneller wirkt, daß außerdem der Verbandwechsel nicht so häufig durchgeführt werden muss, was Patient und Klinikpersonal entlastet und außerdem Kosten spart, und drittens, daß der Verbandwechsel unproblematisch ist, da das Trägermaterial trotz längerer Verweilzeit auf der Wunde nicht mit der Wunde verklebt.

Die Erfindung wird durch die folgenden Beispiele weiter erläutert.

<u>Beispiel 1:</u>
Ein Vliesstoff, hergestellt aus Polytetrafluoräthylen(PTFE)-Folienbändchen, die bereits antiadhäsiv sind, wird von einer Rolle abgewickelt und dabei mit PVP-Jodsalbe mittels einer Breitschlitzdüse beschichtet. Dieses beschichtete Vlies wird zu der gewünschten Länge abgeschnitten und in eine geeignete Packung aus Verbundfolie in bekannter Weise eingesiegelt.

Beispiel 2:

Ein Vliesstoff aus Zellulose wird von einer Rolle abgewickelt
und durch Tauchen in ein Bad von geschmolzener, heißer Vaseline
antiadhäsiv ausgerüstet. Nach dem Erkalten wird dieses Vlies
ebenfalls mittels einer Breitschlitzdüse mit PVP-Jod-Salbe beschichtet und wie in Beispiel 1 angegeben konfektioniert.

Beispiel 3:

Ein grobmaschiges Baumwollgewebe wird durch Tauchen in ein
Silikonölbad antiadhäsiv ausgerüstet und danach mittels einer
Breitschlitzdüse mit einer antibakteriellen Salbe mit dem
Wirkstoff Silbersulfa-diazin beschichtet und wie in Beispiel 1
konfektioniert.


Die Erfindung wird ferner durch die Zeichnungen erläutert.

Fig. 1        ist eine perspektivische Draufsicht auf den
              antiadhäsiven, antimikriobiellen Wundverband
              gemäss der ERfindung;

Fig. 2        ist ein vergrößerter Ausschnitt aus dem anti-
              adhäsiven, antimikriobiellen Wundverband ge-
              mäss der Erfindung.

In den Figuren ist der antiadhäsive, antimikrobielle
Wundverband mit 1 bezeichnet. Das Gewebe oder das Vlies
aus natürlichen oder synthetischen Fasern ist in den Figuren mit 2 bezeichnet. Die antimikriobielle Zubereitung,
z.B. die Salbe oder Creme, beispielsweise eine PVP-Jod-
Salbe oder -Creme , ist in den Figuren mit 3 bezeichnet.

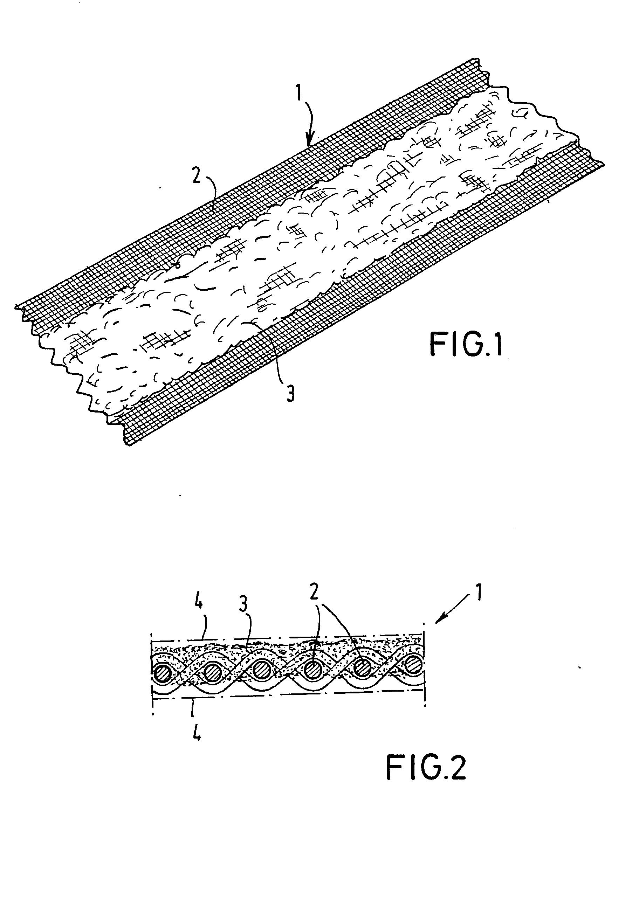

In Fig. 2 ist mit 4 eine Verbundfolie zur Verpackung des
erfindungsgemässen Wundverbands bezeichnet.

Patentansprüche
--------------------------------

1. Antiadhäsiver, antimikrobieller Wundverband auf der Basis von Verbandsmaterial und pharmazeutischen Zubereitungen, dadurch gekennzeichnet, daß ein antiadhäsives oder antiadhäsiv ausgerüstetes Gewebe oder Vlies aus natürlichen oder synthetischen Fasern mit einer antimikrobiellen Zubereitung zur lokalen Therapie beschichtet ist.

2. Wundverband nach Anspruch 1, dadurch gekennzeichnet, daß die antimikrobielle Zubereitung eine antibakterielle Salbe oder Creme ist.

3. Wundverband nach Ansprüchen 1 und 2, dadurch gekennzeichnet, daß die antimikrobielle Zubereitung eine PVP-Jod-Salbe oder -Creme ist.

4. Wundverband nach Ansprüchen 1 - 3, dadurch gekennzeichnet, daß das aus Gewebe, Stoff oder Vliesstoff bestehende Trägermaterial ein Vliesstoff aus Cellulose, ein Vliesstoff aus Polytetrafluoräthylen oder ein grobmaschiges Baumwollgewebe ist.

5. Wundverband nach Ansprüchen 1 - 4, dadurch gekennzeichnet, daß er in eine Verbundfolie eingesiegelt ist.

6. Verfahren zur Herstellung von Wundverbänden nach Ansprüchen 1 - 4, dadurch gekennzeichnet, daß man ein Trägermaterial aus Gewebestoff oder Vliesstoff antiadhäsiv durch Tauchen in ein entsprechendes Bad antiadhäsiv ausrüstet, das Produkt abkühlt und anschließend mit der antimikrobiellen therapeutischen Zubereitung beschichtet bzw. tränkt und ggfls. nach dem Zerschneiden in eine Verbundverpackungsfolie einsiegelt.

0047492

FIG.1

FIG.2